# EUROPEAN PATENT APPLICATION

(11) **EP 2 532 224 A1**
(43) Date of publication of application: **12.12.2012**
(21) Application number: 11169473.3
(22) Date of filing: 10.06.2011
(51) Int. Cl.: A01G 7/04

(54) **Method and means for improving plant productivity through enhancing insect pollination success in plant cultivation**

(71) Applicant: Valoya Oy, 02520 Lapinkylä (FI)
(72) Inventor: Aikala, Lars, 02520 Lapinkylä (FI); Kivimäki, Ilkka, 00150 Helsinki (FI); Kotilainen, Titta, 00610 Helsinki (FI)
(74) Representative: Väänänen, Mikko Kalervo

(57) **Abstract**

The invention relates to the cultivation of insect (840) pollinated plants (710, 711) in a greenhouse (700, 701 and 802) environment. In more particular, the invention relates to a lighting device and a method of illumination designed to enhance insect pollination in plants, such as the tomato. The best mode of the invention is considered to be the use of a LED (101, 102, 103 and 104) lighting device having emission peaks (401, 402, 403, 410 and 510) matching the photosynthetic relative absorption peaks of green plants, and the relative reflectance peaks of flowers of plants being cultivated and the relative sensitivity peaks of the insect's vision being used in the pollination. The inventive lighting device and method reduces insect mortality and increases pollination efficiency, photosynthetic growth and thereby increases the productivity of plant cultivation.

## Description

### TECHNICAL FIELD OF INVENTION

The invention relates to the cultivation of insect pollinated plants in a greenhouse environment. In more particular, the invention relates to a lighting device and a method of illumination designed to enhance insect pollination in plants, such as the tomato.

### BACKGROUND

It is a well known fact that some plants are insect pollinated, the most notable agricultural plant belonging to this category being the tomato. The reproduction of these plants and fruit production depends on the pollination that in natural conditions is conducted by insects and wind. In greenhouse environments mechanical vibrators and hormone treatments of plants have been employed in the prior art to guarantee sufficient levels of pollination. These methods lead to poor quality of fruit. A failed pollination can lead for example to deformed fruit. Introducing insects into greenhouse environments increases crop yield but is quite expensive, as greenhouse gas discharge lights kill the insects quite frequently with their heat, and new insects need to be frequently introduced.

It is also known in the prior art that insect vision is very sensitive to ultraviolet radiation, please see e.g. Limits to salience of ultraviolet: lessons from colour vision in bees and birds, Kevan et al., which is cited here as reference. It is also known that honey bees and bumble bees use motion parallax in their vision, please see Lehrer M. 1998, "Looking all around: honeybees use different cues in different eye regions", Journal of Experimental Biology 201:3275-3292, which document is cited here as reference. As the insect flies over a field of flowers, the flower closer to the insect than its background appears to move faster than the background, thereby creating a relative motion between the flower and the background. In nature the flowers are nearly always moving, typically due to wind, and can thus be easily observed by the insects. In a greenhouse environment there is typically no wind, which makes it difficult for insects to detect the flowers.

The UV feature of insect vision has been utilized by man in the prior art, for example in WO 2009/040528, which is cited here as reference. This publication shows the photo manipulation of insects with Light Emitting Diode (LED) at wavelengths of 353 nanometers (nm), 345-375 nm, 315-400 nm. The insects are photo manipulated towards the light, so that they could be trapped. This device is used in a restaurant environment, so that the customers would not be disturbed by the insects.

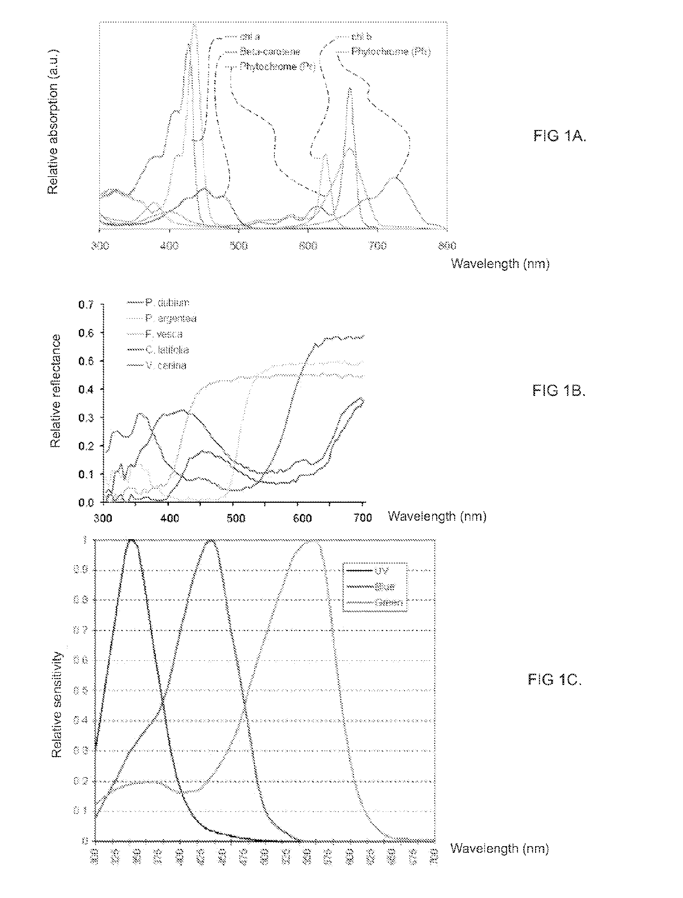

Figure 1A demonstrates the relative absorption spectrum of chlorophyll a and b, Phytochrome Pfr and Pr and beta-carotene in green plants in accordance with the prior art. Further details are explained in WO/2011 033177 of the inventor, which document is cited here as reference. WO/2011 033177 also discloses a LED lighting assembly, which is designed to provide an emission spectrum that has a good photomorphogenetic response in plants, i.e. plants grow fast to the desired shape and size when allowed to enjoy and use light from this lighting assembly for photosynthesis.

It is a well known fact that different flowers reflect light differently. Figure 1B describes the relative reflectance spectrum of flowers of different plants as measured in "Flower colour as advertisement", Chitka L. & Kevan, P.G. (2005), In Dafni, A., Kevan P.G., Husband, B.C. (eds.) Practical Pollination Biology. Enviroquest Ltd., Cambridge, ON, Canada, pp. 157-196 in accordance with the prior art. This document is also cited here as reference. The flowers measured were: *Potentilla argentea,* red *Papaver dubium,* blue *Viola Canina,* violet *Campanula latifolia* and white *Fragaria vesca.*

Figure 1C describes the relative sensitivity spectrum of the insect eye of a honeybee (*Apis mellifera*) as measured in "Flower colour as advertisement", Chitka L. & Kevan, P.G. (2005), In Dafni, A., Kevan P.G., Husband, B.C. (eds.) Practical Pollination Biology. Enviroquest Ltd., Cambridge, ON, Canada, pp. 157-196 in accordance with the prior art. This spectrum confirms that there is a reasonable extent of overlap in high sensitivity bands of insect vision and high reflectance bands of flowers. In fact, the analysis of reflectance spectra of 180 flowers showed that the reflectance peaks match sensitivity peaks of pollinating insect vision in Chittka L. & Menzel R. 1992, "The evolutionary adaptation of flower colours and the insect pollinators' colour vision", Journal of Comparative Physiology A 171: 171-181, which document is cited here as reference.

There are serious disadvantages in the prior art. The aforementioned spectral observations have been used only for trapping insects as pests. Also artificial illumination solutions for plants of the prior art, of which WO/2011 033177 is perhaps the most developed, have addressed the enhancement of only photosynthetic growth of the plants, not their reproduction or fruit production. It is very difficult for pollinating insects to find flowers in greenhouses when it is winter, cloudy weather or otherwise dark, and/or when the air stands still. Realising natural pollination activity that would be as high quality as the natural process, or even higher quality in greenhouse environments is therefore a substantial problem burdening the prior art.

### SUMMARY

It is an object of the invention to solve the aforementioned disadvantages. The invention under study is directed towards a system and a method for effectively illuminating plants so that they achieve the desired photosynthetic growth and are pollinated more frequently by insects.

A further object of the invention is to provide an illumination device that is harmless to the pollinating insects in a greenhouse environment.

In one aspect of the invention the plants are illuminated with a lighting device that comprises LEDs and provides strong emission peaks at wavelengths that coincide with the reflectivity of the flowers being cultivated. This has the effect that the insects can see the flowers better, and therefore find them more easily, which increases the efficiency of pollination by the insects. A further improvement to this aspect is to choose those wavelengths for emission peaks that have a high reflectivity from flowers and/or high sensitivity in the insect vision. The best effect is achieved when the aforementioned high reflectivity and high vision sensitivity coincide. This is the preferred emission wavelength for a pollination enhancing lighting device as it increases the visibility of the flowers in the eyes of the insect maximally.

The wavelengths that are typically suited as emission peaks are for example 348 nm, 375 nm, 435 nm, 533 nm, 538 nm with an error range of ±10 nm. The light device is typically realized with LEDs and/or quantum dots. A *quantum dot* is a semiconductor whose excitons are confined in all three spatial dimensions.

The peak wavelengths may vary depending on the plant species being cultivated and the insect used to cultivate the said plant. For example (*B. terrestris dalmatinus* and *B. terrestris sassaricus*) exhibited spectral peaks at 348 nm, 435 nm and 533 nm and 347 nm, 436 nm and 538 nm, please see Skorupski P., Döring T.F. & Chittka L. 2007, "Photoreceptor spectral sensitivity in island and mainland populations of the bumblebee, Bombus terrestris", Journal of Comparative Physiology A 193: 485-494, which is cited here as reference. *B. impatiens* has its photoreceptor spectral sensitivity peaks at 347 nm, 424 nm and 539 nm, please see Skorupski P. & Chittka L. 2007, "Photoreceptor spectral sensitivity in the bumblebee, Bombus impatiens (Hymenoptera: Apidae)", Plos ONE 5: 1-5, which document is cited here as reference. Any or all of the aforementioned peaks could be used as center wavelengths for light emitter spectral peaks of the invention.

Another aspect of the invention combines the aforementioned light device with a light device that enhances the photosynthetic growth in plants. This light device typically has a peak in the blue emission, low green and/or yellow emission, and a peak in the red and/or far red emission. This maximises photosynthetic growth as it coincides with the absorption peaks of Figure 1A. This emission is also typically produced by LEDs and/or quantum dots.

A horticultural light device in accordance with the invention is characterised in that the said light device is arranged to emit at least one spectral peak at a wavelength that coincides with increased reflectivity of flowers of pollinating plants.

A plant cultivation method in accordance with the invention is characterised in that the pollinating plants are illuminated with a light device emitting at least one spectral peak at a wavelength that coincides with increased reflectivity of flowers of said pollinating plants.

The "coincidence" in this application is understood as the positioning of spectral peaks that aims to maximize the incoming photons from the flower to the insect eye, and ultimately maximize the neurological vision signal that the insect eye generates from the photons. Therefore the peaks do not need to mathematically exactly coincide, in accordance with the invention the peaks need only to coincide to a degree that sufficiently maximizes the neurological vision signal in the insect eye.

The said aforementioned increased reflectivity and/or sensitivity is understood to exceed the 90%-, 80%-, 70%-, 60%-, 50%-, 40%- or 30%- level of maximum of said reflectivity and/or sensitivity in the UV (300-400 nm) to far red (700-800 nm) band. For example if both the reflectivity and the sensitivity would be at 70% level of maximum, the end visual signal detected by the insect would be 0.7*0.7=0,49, i.e. roughly half of the signal that exact coincidence of maximum peaks could produce. This is most probably a sufficient level to assist the insects in pollinating the plants very significantly.

The plant illumination device and method of the invention has the advantage that the LED and/or quantum dot based design is harmless to the pollinating insects. Prior art light devices relying on electric discharge typically attract insects, but also heat to extreme temperatures, killing many pollinating insects that are drawn close to the prior art light device. A further advantage of the invention is that as the insects see the flowers better; pollination efficiency is increased, leading to more enhanced reproduction by the plants, higher volume and quality fruit production and an increase in crop. The better survival rate and improved vision have a synergistic added advantage: the insects are known to be capable of learning to operate in different illumination wavelengths and conditions. However, if the insects are killed by hot lamps very early on, no learning will have taken place. A light device that is not lethal to insects also adds to improved pollination by its effect of allowing more educated insects to pollinate the plants more effectively than ever before. The light device provides also for better rested insects as the insects find to their nest easier with the inventive light solution than without it. As the insects are capable of learning, it is possible that the insects can work quite effectively in illumination conditions where the peak wavelength of the illumination is not at maximum sensitivity, when they are provided with the chance to adjust to the lighting conditions.

LEDs of the invention provide also an improvement to current solution using HPS and HID (High Intensity Discharge) due to their capability of easy spectrum in-situ tuning, e.g. UV LEDs can only be turned on during a pollination event, i.e. when pollinating insects are present near the plants. When turned off UV light does not assist harmful insects, such as pests, to find plants. This is not possible in HPS and HID lamps of the prior art. Producing UV light also consumes more energy and therefore it is beneficial to use that spectrum off when not used.

An even further advantage of the invention is that as the light device of the invention enhances both reproduction and growth, this enhancement has a synergistic improvement in crop levels that goes beyond the levels that could be achieved by using either one lighting solution individually or separately. Even further, the invention has the advantage that fruit production becomes possible in polar regions, cloudy weather conditions and winter seasonal times that have been impossible to use for fruit production before due to low natural light levels.

In addition and with reference to the aforementioned advantage accruing embodiments, the best mode of the invention is considered to be the use of a LED lighting device having emission peaks matching the photosynthetic relative absorption peaks of green plants, and the relative reflectance peaks of flowers of plants being cultivated and the relative sensitivity peaks of the insect's vision being used in the pollination.

### BRIEF DESCRIPTION OF THE DRAWINGS

In the following the invention will be described in greater detail with reference to exemplary embodiments in accordance with the accompanying drawings, in which
Figure 1A demonstrates the relative absorption spectrum of chlorophyll a and b, Phytochrome Pfr and Pr and beta-carotene in green plants in accordance with the prior art.
Figure 1B describes the relative reflectance spectrum of flowers of different plants as measured in "Flower colour as advertisement", Chitka L. & Kevan, P.G. (2005), In Dafni, A., Kevan P.G., Husband, B.C. (eds.) Practical Pollination Biology. Enviroquest Ltd., Cambridge, ON, Canada, pp. 157-196 in accordance with the prior art.
Figure 1C describes the relative sensitivity spectrum of the insect eye of a honeybee (*Apis mellifera*) in accordance with the prior art as measured in "Flower colour as advertisement", Chitka L. & Kevan, P.G. (2005), In Dafni, A., Kevan P.G., Husband, B.C. (eds.) Practical Pollination Biology, Enviroquest Ltd., Cambridge, ON, Canada, pp. 157-196.
Figure 2 demonstrates an embodiment 20 of the insect pollination enhancing method in accordance with the invention as a flow diagram.
Figure 3A demonstrates an embodiment 30 of the light emitting device in accordance with the invention as a block diagram.
Figure 3B demonstrates an embodiment 31 of the light emitting device utilising wavelength up-conversion in accordance with the invention as a block diagram.
Figure 3C demonstrates an embodiment 32 of the light emitting device utilising quantum dots in accordance with the invention as a block diagram.
Figure 4 demonstrates an embodiment 40 of the emission spectrum of the light emitting device arranged to enhance insect pollination in accordance with the invention.
Figure 5 demonstrates an embodiment 50 of the emission spectrum of the light emitting device arranged to enhance insect pollination in accordance with the invention.
Figure 6 demonstrates an embodiment 60 of the emission spectrum of the light emitting device arranged to enhance insect pollination in accordance with the invention.
Figure 7 demonstrates an embodiment 70 of the light emitting device arranged to enhance insect pollination in a greenhouse environment in accordance with the invention as a block diagram.
Figure 8 demonstrates an embodiment 80 of the light emitting device arranged to enhance insect pollination and plant cultivation in an urban basement environment in accordance with the invention as a block diagram.

Some of the embodiments are described in the dependent claims.

### DETAILED DESCRIPTION OF EMBODIMENTS

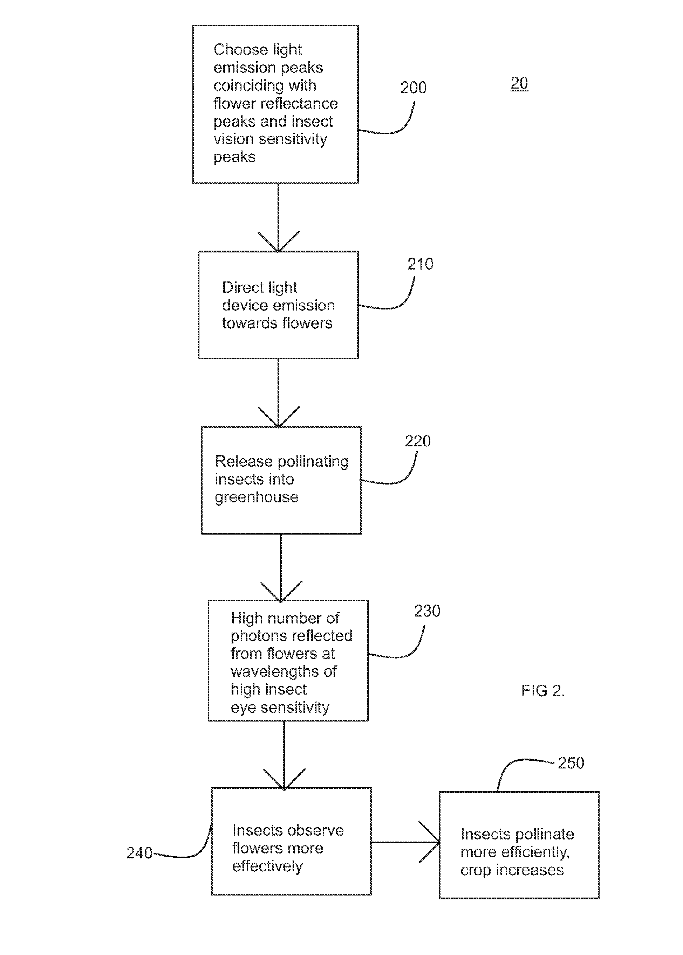

Figure 2 shows the method of the invention as a flow diagram. In phase 200 the light emission peaks are chosen. The emission peaks should have wavelengths that coincide with the reflectivity peaks of flowers of plants being pollinated and cultivated. The reflectance curves were shown in Figure 1B for a number of exemplary flowering plants. Furthermore the spectral peak should be emitted at a wavelength that coincides with increased photoreception sensitivity of insect vision. The exemplary sensitivity curves were shown in Figure 1C. These spectral peaks occur roughly at any of the following wavelengths: 348 nm, 375 nm, 435 nm, 538 nm with an error range of ±10 nm in accordance with the invention. The peak wavelengths will of course vary depending on the plant species being cultivated and the insect used to cultivate the said plant, and it is in accordance with the invention to choose one or more specific emission peaks for each insect pollinator-flower pair.

From the Figures 1B and 1C it can be deduced that the greatest coincidence of spectral maxima appears to happen quite close to the insect vision sensitivity maximas.

Preferably, the said increased reflectivity and/or sensitivity exceeds the I/√2 of maximum of said reflectivity and/or sensitivity in the UV to far red band in some embodiments of the invention. In some embodiments at coincidence wavelength the reflectivity and/or sensitivity exceeds the 90%-, 80%-, 70%-, 60%-, 50%-, 40%- or 30%- level of maximum of said reflectivity and/or sensitivity in the UV (300-400 nm) to far red (700-800 nm) band.

The light device is typically arranged to comprise at least one LED and/or quantum dot, or be composed entirely ofLEDs and/or quantum dots. This design choice allows for greater spectral design freedom needed in optimizing the emission peaks to match the aforementioned flower reflectivity and insect eye sensitivity spectral peaks. Furthermore, this emitter technology has the added effect that these lighting devices do not heat to levels that are lethal to the pollinating insects.

The light device and the method is/are typically used in a greenhouse and/or indoor environment where insect pollinated plants are cultivated, but can also be used outdoors. In phase 210 the light emission is directed towards flowers in the greenhouse. In phase 220 the pollinating insects are released into the greenhouse. Typically these insects are honeybees and/or bumblebees.

In phase 230 a high number of photons are reflected from flowers at wavelengths of high insect vision sensitivity. This creates an environment where the insects observe the flowers extremely easily and therefore discover pollination targets as effectively as possible in phase 240.

The increased observability of pollination targets by the insects leads to enhanced pollination and thereby the crop being cultivated increases in yield substantially in phase 250. This method is preferably used in combination with a light device and method that optimizes photosynthetic growth. Preferably the light device used in combination with method 20 is a horticultural lighting fixture comprising at least one Light Emitting Diode (LED) having a first spectral characteristics including a peak in the wavelength range from 600 to 700 nm and arranged to exhibit a full width at half maximum of at least 50 nm or more and a second spectral characteristics with a maximum of 50 nm full width at half maximum and arranged to exhibit a peak wavelength in the range from 440 to 500 nm.

Furthermore in some embodiments of the invention at least a part or the whole of the emission at wavelengths of 500-600 nm is minimized and/or omitted and/or reduced below the intensity in 400-500 nm band and below the intensity in 600-700 nm band. As Figure 1A shows the photosynthetic absorption is quite low in this band. Also in some embodiments the emission spectrum comprises far red radiation (700-800 nm), which has been observed by the applicant to enhance biomass growth in plants as a surprise effect.

The emission can be achieved by powering LEDs and/or quantum dots electrically and/or by optical up-conversion in accordance with the invention. In optical up-conversion short wavelength radiation is absorbed and then optically re-emitted at a longer wavelength. Quantum dots and/or phosphorus can be used to realize the wavelength up-conversion of the invention. In one embodiment that is especially preferable the far red radiation (700-800 nm) is produced by for example europiumcerium co-doped BaₓSr_{y}ZnS₃ phosphors and/or cerium doped lanthanide oxide sulfides. These phosphor and sulfide types have emission peak maxima between 650-700 nm wavelength region and exhibit also broad (50-200 nm) full width at half maximum and therefore also produce light emission at higher wavelength, i.e., above 700 nm wavelength range.

In one embodiment, all or part of the emission at a frequency of 600-800 nm is generated using a whole or partial wavelength up-conversion of the LED chip radiation power.

It should also further be noted that the embodiment 20 can be readily permuted and/or combined with any of the embodiments 30, 31, 32, 40, 50, 60, 70, 71 and/or 80 in accordance with the invention.

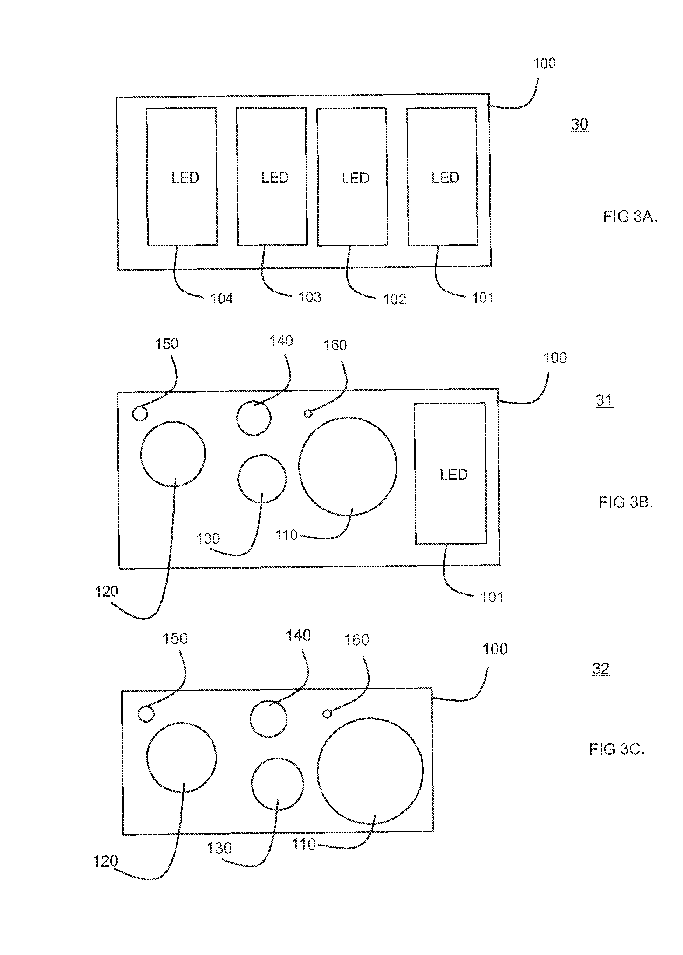

Figure 3A shows the embodiment where the light device is realised on a chip 100 using LEDs 101, 102, 103, 104 only. Naturally any number of LEDs at different wavelengths and FWHM's (Full Width at Half Maximum) can be used in accordance with the invention. One or more wavelength up-converters can also be used in accordance with the invention.

Preferably the at least one LED 101, 102, 103, 104 produces an emission spectrum that has the peaks at wavelengths that coincide with high photosynthetic absorption, high flower reflectivity and/or high insect vision sensitivity as explained before in some embodiments.

It should also further be noted that the embodiment 30 can be readily permuted and/or combined with any of the embodiments 20, 31, 32, 40, 50, 60, 70, 71 and/or 80 in accordance with the invention.

Furthermore any light emitter design from EP 11158698.8, of the inventor and applicant can be combined with embodiment 30. This document is cited here as reference.

Figure 3B shows an embodiment where at least one LED and at least one quantum dot are used in combination. In this example there is a LED emitter 101 and quantum dots 110, 120, 130, 140, 150, 160.

The LED can typically be only driven by electric power. The quantum dots can be driven by electric power to produce light emission, but in some embodiments all or some quantum dots can be used as wavelength up-converters of absorbed optical radiation also. Preferably some quantum dots are of different size in some embodiments, as a different dot diameter implies a different emission spectrum.

Preferably the at least one LED 101 and at least one quantum dot 110, 120, 130, 140, 150, 160 produce an emission spectrum that produces the peaks at wavelengths that coincide with high photosynthetic absorption, high flower reflectivity and/or high insect vision sensitivity as explained before in some embodiments.

All or some of the quantum dots 110, 120, 130, 140, 150 and 160 are typically manufactured from any of the following alloys: cadmium selenide, cadmium sulphide, indium arsenide, indium phosphide and/or cadmium selenide sulphide in some embodiments. In one special exemplary embodiment of the invention CdSe-ZnS (core-shell) quantum dot nano particles with average particle size of 6.6 nm with approximately +/- 0.5 nm particle size distribution were mixed with a two component silicone encapsulant resin. The mixing ratio was 0.2 w-% of nano particles in the silicone resin. The resin containing nano particles were dispensed as encapsulant into a plastic leaded chip carrier (PLCC) consisting a InGaN light emitting diode in the PLCC cavity. The light emitting diodes was determined to have electroluminescent emission at 450 nm wavelength range.

The InGaN containing PLCC package with nano particles containing encapsulant material was connected to a DC voltage power source with forward voltage of 3.2 V and current of 350 mA. In some embodiments the LED has a higher or lower current, for example 450 mA LEDs are now being implemented in one other alternative design by the applicant. The device optical emission spectrum was characterized to result in two emission peaks one at 450 nm wavelength range and the second at the 660 nm wavelength range. The 660 nm wavelength range emission peak's full width at half maximum was observed to be over approximately 60 nm. The intensity ratios of the 450 nm and 660 nm peaks were 0.5:1. The aforementioned experiment has been conducted by the applicant. It is in accordance with the invention to produce several quantum dots as described above, some of different sizes. These quantum dots, one or many quantum dots may be driven with electric current/voltage from a power source or the said one or many quantum dots may be driven by optical excitation or both optical excitation and electric current/voltage from a power source in accordance with the invention.

It should also further be noted that the embodiment 31 can be readily permuted and/or combined with any of the embodiments 20, 30, 32, 40, 50, 60, 70, 71 and/or 80 in accordance with the invention.

Furthermore any light emitter design from EP11158648.3, of the inventor and applicant can be combined with embodiment 31. This document is cited here as reference.

Figure 3C shows the embodiment where the light emitter chip features only quantum dots. Preferably the quantum dots have a size distribution that produces an emission spectrum that produces the peaks at wavelengths that coincide with high photosynthetic absorption, high flower reflectivity and/or high insect vision sensitivity as explained before in some embodiments.

It should also further be noted that the embodiment 32 can be readily permuted and/or combined with any of the embodiments 20, 30, 31, 40, 50, 60, 70, 71 and/or 80 in accordance with the invention.

Furthermore any light emitter design from EP11158693.9, of the inventor and applicant can be combined with embodiment 32. This document is cited here as reference.

It should be noted that any of the embodiments 30, 31, 32 may be used to produce a broad UV component in some embodiments of the invention.

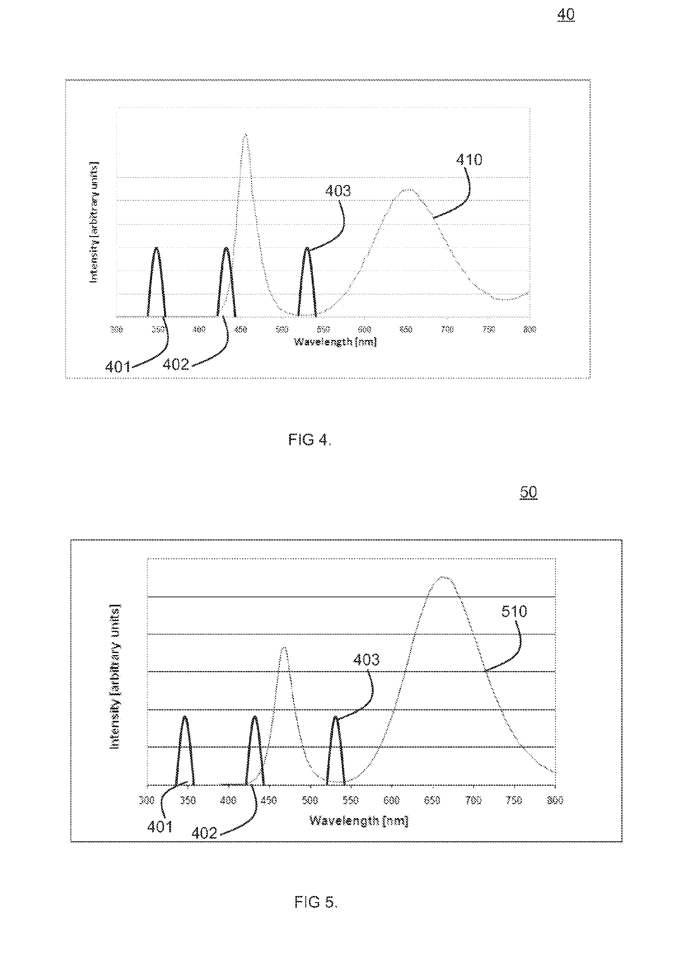

Figure 4 shows an exemplary spectral diagram 40 that could be produced by the method and light device of the invention. The spectral feature 410 in grey thin line is arranged to maximise photosynthetic absorption per watt spent and typically this feature is produced by a blue LED with a wavelength up-conversion phosphor and/or quantum dot. In other embodiments this feature is produced by two LEDs and/or quantum dots.

The spectral features 401, 402, 403 in thick black are designed to illuminate the flowers in the plants to insects, making them maximally observable to insects. These features are typically produced by a LED and/or a quantum dot powered by electric current. In some embodiments filters, such as band-pass filters can be used with LEDs and/or quantum dots to produce the spectral features 401, 402, 403.

At least one spectral feature 401, 402, 403 should have a relative intensity level sufficient to highlight the flower from the background. In one preferred embodiment the relative intensity level is such that the intensity is doubled by the spectral features 401, 402, 403 in the respective bands of the spectral features. In another preferred embodiment, the relative intensity level is such that the intensity is increased to ten times higher or more by the spectral features 401, 402, 403 in the respective bands of the spectral features, thus providing logarithmic amplification.

It should also further be noted that the embodiment 40 can be readily permuted and/or combined with any of the embodiments 20, 30, 31, 32, 50, 60, 70, 71 and/or 80 in accordance with the invention.

Figure 5 resembles Figure 4 otherwise, except that the plant growth enhancing photosynthetic spectral feature 510 is redder than the corresponding photosynthetic spectral feature 410 in figure 4 embodiment 40. The relative intensity of pollination enhancing spectral features 401, 402, 403 can be at any level in accordance with the invention.

It should also further be noted that the embodiment 50 can be readily permuted and/or combined with any of the embodiments 20, 30, 31, 32, 40, 60, 70, 71 and/or 80 in accordance with the invention.

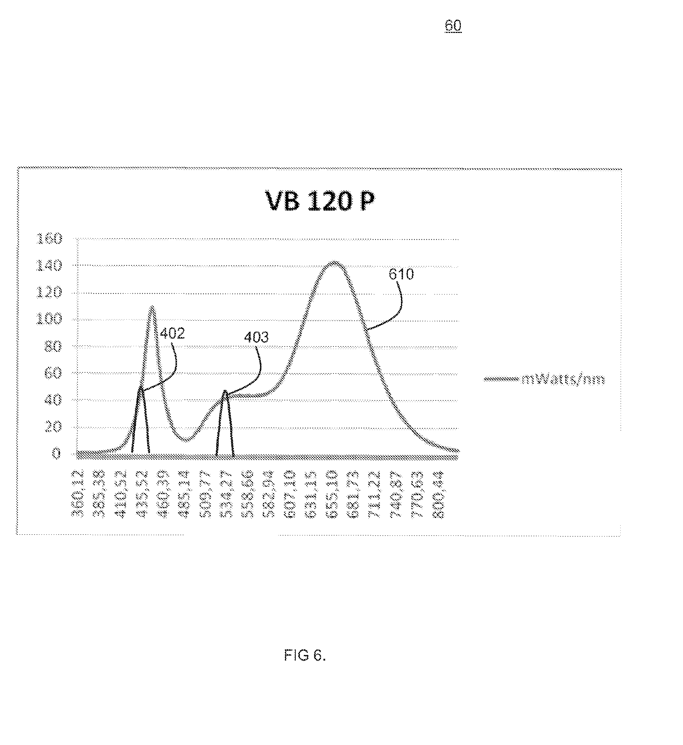

Figure 6 displays the embodiment 60 that has a photosynthetic spectral feature 610 which has been tested in greenhouses by the applicant. This spectral feature was particularly successful in conditions of limited natural light. In embodiment 60 this photosynthetic spectral feature is complemented by the pollination enhancing spectral features of 402 and 403.

It should also further be noted that the embodiment 60 can be readily permuted and/or combined with any of the embodiments 20, 30, 31, 32, 40, 50, 70 and/or 80 in accordance with the invention.

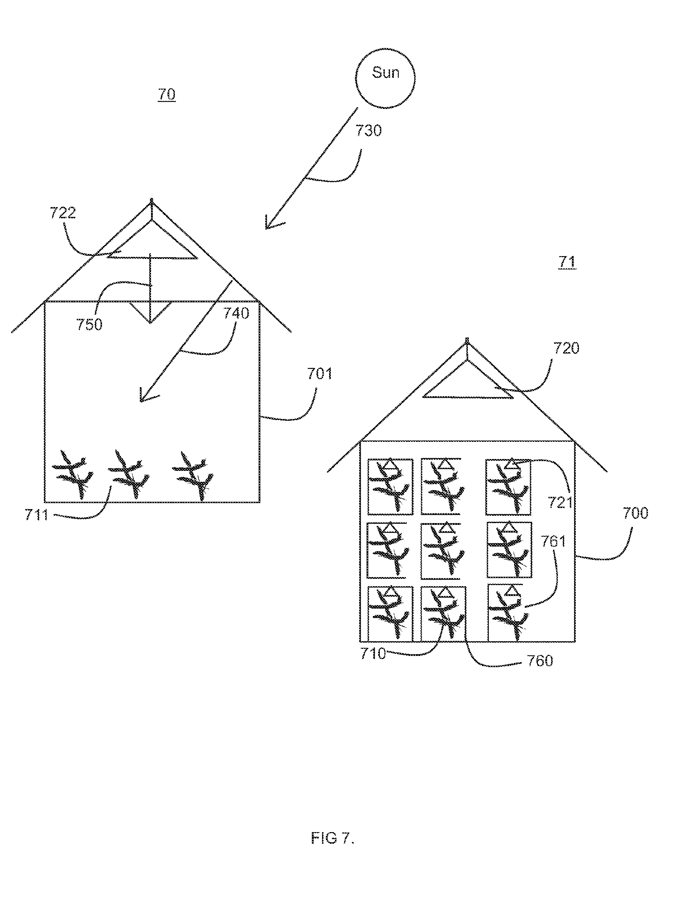

Figure 7 shows different use configurations of the lighting device of the invention in a greenhouse environment. In embodiment 70 at least one plant is on the greenhouse floor and the lighting device shines light on one or more plants 711. Typically the greenhouse 701 has transparent walls, so the sunlight 730 emerging through the wall 740 will be similar to the solar spectrum minus the filtrating effect of the wall material. The spectrum 750, which as explained before included peaks at wavelengths that coincide with high photosynthetic absorption, high flower reflectivity and/or high insect vision sensitivity, are optimised to still contain those peaks in the presence of complementing natural solar light in accordance with the invention.

It should also further be noted that the embodiment 70 can be readily permuted and/or combined with any of the embodiments 20, 30, 31, 32, 40, 50, 60, 71 and/or 80 in accordance with the invention.

In embodiment 71 the plants are arranged in shelves in open and closed growth chambers to save space. Naturally the inventive lighting device can be used to illuminate all or some plants collectively, as the lighting device 720 attached to the roof, or small lighting devices of the invention can be attached on the shelves, or in growth chambers, to illuminate plants to the insects more locally.

It should also further be noted that the embodiment 71 can be readily permuted and/or combined with any of the embodiments 20, 30, 31, 32, 40, 50, 60, 70 and/or 80 in accordance with the invention.

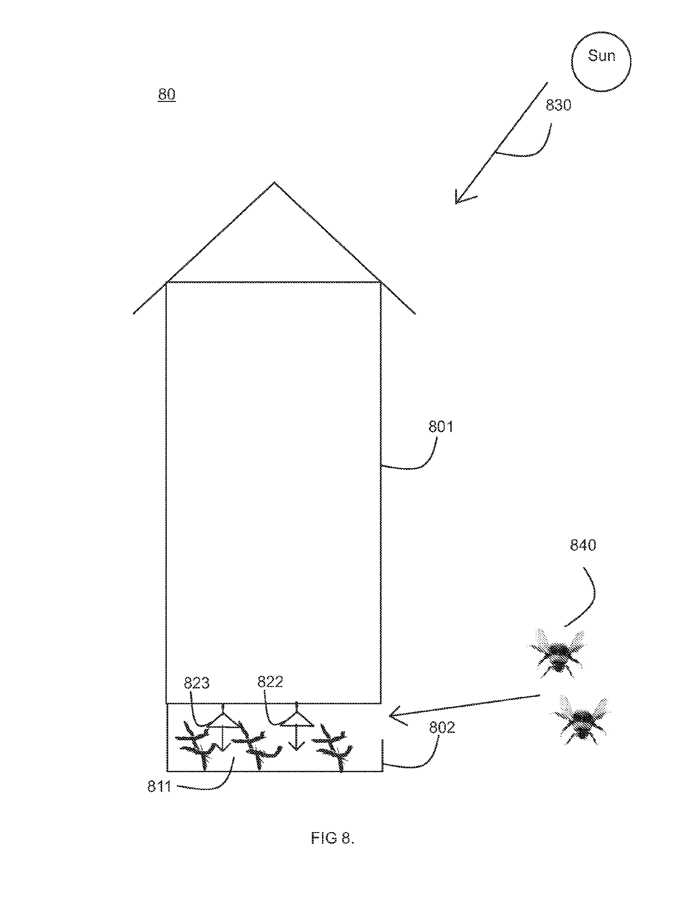

Figure 8 shows a use embodiment of the invention suitable for a skyscraper in a big city. People typically prefer to live on higher floors in an urban environment, because these floors are further from the street noise and have more natural light, as evidenced by property prices in many European and US cities. In this embodiment the lower floors of the skyscraper are utilised for local food production by having at least one plant 811 in a lower floor, where at least one lighting device 822, 823 of the invention is/are used to illuminate at least one plant. The at least one plant 811 is an insect pollinated plant, and insects 840 are lured into the lower floor and the plants 811 by the spectral features emitted by the light devices 822, 823 that attract the insects and display the flowers in the at least one plant 811 to the insects more effectively. This leads to high efficiency pollination and high crop levels per unit area near the consumer, where space is at a premium.

It should also further be noted that the embodiment 80 can be readily permuted and/or combined with any of the embodiments 20, 30, 31, 32, 40, 50, 60, 70 and/or 71 in accordance with the invention.

In some embodiments of the invention one or more spectral peaks are arranged to be emitted in the 340-440 nm band. It should be noted that in any of the aforementioned embodiments it is also in accordance with the invention to provide a broad UV spectrum, providing broader usability, as the reflectivity of the light on the various surfaces is better in general. The broader UV component may be used to replace one, more or all spectral peaks or complement them in accordance with the invention. Using a quantum dot LED it is possible to produce a broad electroluminescence spectrum at UV wavelengths. In all of the aforementioned embodiments it is possible to provide for a feature where the photosynthetic illumination spectral component and the pollination enhancing spectral component are controlled separately, i.e. one, the other or both may be on or off as needed. It is also in accordance with the invention to provide means for adjusting the relative emission intensities of the said two components.

The invention has been explained above with reference to the aforementioned embodiments and several commercial and industrial advantages have been demonstrated. The methods and arrangements of the invention allow lower insect mortality as the LED and/or quantum dot based design is harmless to the pollinating insects. Prior art light devices relying on electric discharge typically attracted insects, but also heated to extreme temperatures, killing many pollinating insects that were drawn close to the prior art light device. A further advantage of the invention is that as the insects see the flowers better, pollination efficiency is increased, leading to more enhanced reproduction by the plants and an increase in crop.

The better survival rate and improved vision have a synergistic added advantage: the insects are known to be capable of learning to operate in different illumination conditions and wavelengths. However, if the insects are killed by hot lamps very early on, no learning will have taken place. A light device that is not lethal to insects also adds to improved pollination by its effect of allowing more educated insects to pollinate the plants more effectively than ever before. The light device provides also for better rested insects as the insects find to their nest easier with the inventive light solution than without it. As the insects are capable of learning, it is possible that the insects can work quite effectively in illumination conditions where the peak wavelength of the illumination is not at maximum sensitivity, when they are provided with the chance to adjust to the lighting conditions.

LEDs of the invention provide also an improvement to current solution using HPS and HID due to their capability of easy spectrum in-situ tuning, e.g. UV LEDs can only be turned on during a pollination event, i.e. when pollinating insects are present near the plants. When turned off UV light does not assist harmful insects, such as pests, to find plants. This is not possible in HPS and HID lamps of the prior art. Producing UV light also consumes more energy and therefore it is beneficial to have that spectrum component off when not being used.

An even further advantage of the invention is that the light device of the invention enhances any or all of the following: fruit production, plant reproduction and/or plant growth. This enhancement has a synergistic improvement in crop levels that goes beyond the levels that could be achieved by using either one illumination solution individually or separately, and it goes beyond the sum of the individual effects. More green plant growth provides the basis for bigger and higher quality fruit, the better developed fruit increases the prospects of success for the next greenhouse plant generation, more plant reproduction produces more fruit, which more fruit is better supported by the photosynthetically stronger plant growth. It is with these synergistic improvements that the invention alleviates the global problem of hunger.

The invention has been explained above with reference to the aforementioned embodiments. However, it is clear that the invention is not only restricted to these embodiments, but comprises all possible embodiments within the spirit and scope of the inventive thought and the following patent claims.

### REFERENCES

"Flower colour as advertisement", Chitka L. & Kevan, P.G. (2005), In Dafni, A., Kevan P.G., Husband, B.C. (eds.) Practical Pollination Biology. Enviroquest Ltd., Cambridge, ON, Canada, pp. 157-196.
Chittka L. & Menzel R. 1992, "The evolutionary adaptation of flower colours and the insect pollinators' colour vision", Journal of Comparative Physiology A 171: 171-181.
Lehrer M. 1998, "Looking all around: honeybees use different cues in different eye regions", Journal of Experimental Biology 201:3275-3292.
"Limits to salience of ultraviolet: lessons from colour vision in bees and birds", Kevan P.G. et al., Journal of Experimental Biology 204, p. 2571-2580 (2001).
Skorupski P., Döring T.F. & Chittka L. 2007, "Photoreceptor spectral sensitivity in island and mainland populations of the bumblebee, Bombus terrestris", Journal of Comparative Physiology A 193: 485-494.
Skorupski P. & Chittka L. 2007, "Photoreceptor spectral sensitivity in the bumblebee, Bombus impatiens (Hymenoptera: Apidae)", Plos ONE 5: 1-5.
EP 11158698.8, "Plant Illumination Device and Method for dark growth chambers", L. Aikala and I. Kivimäki.
EP 11158648.3, "Method and means for enhancing greenhouse lights", L. Aikala and I. Kivimäki.
EP11158693.9, "Plant Illumination Device and Method", L. Aikala and I. Kivimäki.
WO/2011 033177, Lighting Assembly, L. Aikala.
WO 2009/040528, "An Insect Trap", Willcox J.C. and Weaver J.M.

## Claims

1. A horticultural light device, **characterised in that,** said light device is arranged to emit at least one spectral peak (401, 402, and 403) at a wavelength that coincides with increased reflectivity of flowers of pollinating plants (710, 711).

2. A light device as claimed in claim 1, **characterised in that,** said light device is arranged to emit at least one spectral peak (401, 402, and 403) at a wavelength that coincides with increased photoreception sensitivity of insect (840) vision.

3. A light device as claimed in claim 1 or 2, **characterised in that,** the said increased reflectivity and/or sensitivity is understood to exceed the 90%-, 80%-, 70%-, 60%-, 50%-, 40%- or 30%- level of maximum of said reflectivity and/or sensitivity in the UV (300-400 nm) to far red (700-800 nm) band.

4. A light device as claimed in claim 1, **characterised in that,** said light device is arranged into a greenhouse (700, 701, and 802) that is arranged to cultivate insect pollinated plants (710, 711).

5. A light device as claimed in claim 1, **characterised in that,** said light device is arranged with at least one LED (101, 102, 103 and 104) and/or quantum dot (110, 120, 130, 140, 150 and 160).

6. A light device as claimed in claim 1, **characterised in that,** at least one spectral peak is arranged to occur at any of the following wavelengths: 348 nm, 424 nm, 435 nm, 533 nm, 538 nm with an error range of ± 10 nm and/or the lighting device is arranged to emit a broad and flat spectral peak resulting in a broad UV continuum component.

7. A light device as claimed in claim 1, **characterised in that,** the light device is a horticultural lighting fixture comprising at least one Light Emitting Diode (LED) (101, 102, 103 and 104) having
a) first spectral characteristics including a peak in the wavelength range from 600 to 700 nm and arranged to exhibit a full width at half maximum of at least 50 nm or more,
b) second spectral characteristics with a maximum of 50 nm full width at half maximum and arranged to exhibit a peak wavelength in the range from 440 to 500 nm (410, 510 and 610).

8. A light device as claimed in claim 1, **characterised in that,** at least a part or the whole of the emission at wavelengths of 500-600 nm is arranged to be minimized and/or omitted and/or to be reduced below the intensity in 400-500 nm band and below the intensity in 600-700 nm band.

9. A light device as claimed in claim 1, **characterised in that,** the emission spectrum (40, 50 and 60) is arranged to comprise far red radiation (700-800 nm).

10. A light device as claimed in claim 5, **characterised in that,** all or part of the emission at the wavelength band of 600-800 nm is arranged to be generated using a whole or partial wavelength up-conversion of the LED (101, 102, 103 and 104) chip radiation power.

11. A light device as claimed in claim 1, **characterised in that,** at least one said spectral peak is arranged to be turned on or off without influencing the emission of other spectral components in said lighting device.

12. A plant cultivation method, **characterised in that,** plants (710, 711) are illuminated with a light device emitting at least one spectral peak (401, 402 and 403) at a wavelength that coincides with increased reflectivity of flowers of said pollinating plants.

13. A plant cultivation method as claimed in claim 12, **characterised in that,** said light device is arranged to emit at least one spectral peak (401, 402 and 403) at a wavelength that coincides with increased photoreception sensitivity of insect vision.

14. A plant cultivation method as claimed in claim 12 or 13, **characterised in that,** the said increased reflectivity and/or sensitivity is understood to exceed the 90%-, 80%-, 70%-, 60%-, 50%-, 40%- or 30%- level of maximum of said reflectivity and/or sensitivity in the UV (300-400 nm) to far red (700-800 nm) band.

15. A plant cultivation method as claimed in claim 12, **characterised in that,** said light device is used in a greenhouse (700, 701 and 802) where insect pollinated plants (710, 711) are cultivated.

16. A plant cultivation method as claimed in claim 12, **characterised in that,** said light device comprises at least one LED (101, 102, 103 and 104) and/or quantum dot (110, 120, 130, 140, 150 and 160).

17. A plant cultivation method as claimed in claim 12, **characterised in that,** at least one spectral peak occurs at any of the following wavelengths: 348 nm, 424 nm, 435 nm, 533 nm, 538 nm with an error range of ± 10 nm and/or and/or the lighting device emits a broad and flat spectral peak resulting in a broad UV continuum component.

18. A plant cultivation method as claimed in claim 12, **characterised in that,** the light device is a horticultural lighting fixture comprising at least one Light Emitting Diode (LED) (101, 102, 103 and 104) having
c) first spectral characteristics including a peak in the wavelength range from 600 to 700 nm and exhibiting a full width at half maximum of at least 50 nm or more,
d) second spectral characteristics with a maximum of 50 nm full width at half maximum and exhibiting a peak wavelength in the range from 440 to 500 nm (410, 510 and 610).

19. A plant cultivation method as claimed in claim 12, **characterised in that,** at least a part or the whole of the emission at wavelengths of 500-600 nm is minimized and/or omitted and/or reduced below the intensity in 400-500 nm band and below the intensity in 600-700 nm band.

20. A plant cultivation method as claimed in claim 12, **characterised in that,** the emission spectrum (40, 50 and 60) comprises far red radiation (700-800 nm).

21. A plant cultivation method as claimed in claim 16, **characterised in that,** all or a part of the emission at the wavelength band of 600-800 nm is generated using a whole or partial wavelength up-conversion of the LED (101, 102, 103 and 104) chip radiation power.

22. A plant cultivation method as claimed in claim 12, **characterised in that,** at least one said spectral peak is arranged to be turned on or off without influencing the emission of other spectral components in said lighting device.

## Amended claims

### Amended claims in accordance with Rule 137(2) EPC.

**1.** A horticultural light device arranged to emit at least one spectral peak (401, 402, and 403) at a wavelength that coincides with increased reflectivity of flowers of pollinating plants (710, 711), **characterised in that**, and said light device is arranged to emit at least one spectral peak (401, 402, and 403) at a wavelength that coincides with increased photoreception sensitivity of insect (840) vision.

**2.** A light device as claimed in claim 1, **characterised in that**, the said increased reflectivity and/or sensitivity is understood to exceed the 90%-, 80%-, 70%-, 60%-, 50%-, 40%- or 30%- level of maximum of said reflectivity and/or sensitivity in the UV (300-400 nm) to far red (700-800 nm) band.

**3.** A light device as claimed in claim 1, **characterised in that**, said light device is arranged into a greenhouse (700, 701, and 802) that is arranged to cultivate insect pollinated plants (710, 711).

**4.** A light device as claimed in claim 1, **characterised in that**, said light device is arranged with at least one LED (101, 102, 103 and 104) and/or quantum dot (110, 120, 130, 140, 150 and 160).

**5.** A light device as claimed in claim 1, **characterised in that**, at least one spectral peak is arranged to occur at any of the following wavelengths: 348 nm, 424 nm, 435 nm, 533 nm, 538 nm with an error range of ± 10 nm and/or the lighting device is arranged to emit a broad and flat spectral peak resulting in a broad UV continuum component.

**6.** A light device as claimed in claim 1, **characterised in that**, the light device is a horticultural lighting fixture comprising at least one Light Emitting Diode (LED) (101, 102, 103 and 104) having
a) first spectral characteristics including a peak in the wavelength range from 600 to 700 nm and arranged to exhibit a full width at half maximum of at least 50 nm or more,
b) second spectral characteristics with a maximum of 50 nm full width at half maximum and arranged to exhibit a peak wavelength in the range from 440 to 500 nm (410, 510 and 610).

**7.** A light device as claimed in claim 1, **characterised in that**, at least a part or the whole of the emission at wavelengths of 500-600 nm is arranged to be minimized and/or omitted and/or to be reduced below the intensity in 400-500 nm band and below the intensity in 600-700 nm band.

**8.** A light device as claimed in claim 1, **characterised in that**, the emission spectrum (40, 50 and 60) is arranged to comprise far red radiation (700-800 nm).

**9.** A light device as claimed in claim 4, **characterised in that**, all or part of the emission at the wavelength band of 600-800 nm is arranged to be generated using a whole or partial wavelength up-conversion of the LED (101, 102, 103 and 104) chip radiation power.

**10.** A light device as claimed in claim 1, **characterised in that**, at least one said spectral peak is arranged to be turned on or off without influencing the emission of other spectral components in said lighting device.

**11.** A plant cultivation method, comprising the following steps: plants (710, 711) are illuminated with a light device emitting at least one spectral peak (401, 402 and 403) at a wavelength that coincides with increased reflectivity of flowers of said pollinating plants, **characterised in that**, said light device is arranged to emit at least one spectral peak (401, 402 and 403) at a wavelength that coincides with increased photoreception sensitivity of insect vision.

**12.** A plant cultivation method as claimed in claim 11, **characterised in that**, the said increased reflectivity and/or sensitivity is understood to exceed the 90%-, 80%-, 70%-, 60%-, 50%-, 40%- or 30%- level of maximum of said reflectivity and/or sensitivity in the UV (300-400 nm) to far red (700-800 nm) band.

**13.** A plant cultivation method as claimed in claim 11, **characterised in that**, said light device is used in a greenhouse (700, 701 and 802) where insect pollinated plants (710, 711) are cultivated.

**14.** A plant cultivation method as claimed in claim 11, **characterised in that**, said light device comprises at least one LED (101, 102, 103 and 104) and/or quantum dot (110, 120, 130, 140, 150 and 160).

**15.** A plant cultivation method as claimed in claim 11, **characterised in that**, at least one spectral peak occurs at any of the following wavelengths: 348 nm, 424 nm, 435 nm, 533 nm, 538 nm with an error range off ± 10 nm and/or and/or the lighting device emits a broad and flat spectral peak resulting in a broad UV continuum component.

**16.** A plant cultivation method as claimed in claim 11, **characterised in that**, the light device is a horticultural lighting fixture comprising at least one Light Emitting Diode (LED) (101, 102, 103 and 104) having
c) first spectral characteristics including a peak in the wavelength range from 600 to 700 nm and exhibiting a full width at half maximum of at least 50 nm or more,
d) second spectral characteristics with a maximum of 50 nm full width at half maximum and exhibiting a peak wavelength in the range from 440 to 500 nm (410, 510 and 610).

**17.** A plant cultivation method as claimed in claim 11, **characterised in that**, at least a part or the whole of the emission at wavelengths of 500-600 nm is minimized and/or omitted and/or reduced below the intensity in 400-500 nm band and below the intensity in 600-700 nm band.

**18.** A plant cultivation method as claimed in claim 11, **characterised in that**, the emission spectrum (40, 50 and 60) comprises far red radiation (700-800 nm).

**19.** A plant cultivation method as claimed in claim 14, **characterised in that**, all or a part of the emission at the wavelength band of 600-800 nm is generated using a whole or partial wavelength up-conversion of the LED (101, 102, 103 and 104) chip radiation power.

**20.** A plant cultivation method as claimed in claim 11, **characterised in that**, at least one said spectral peak is arranged to be turned on or off without influencing the emission of other spectral components in said lighting device.
